(19) Europäisches Patentamt
European Patent Office
Office européen des brevets

(11) Publication number: **0 118 205**
**B1**

(12) # EUROPEAN PATENT SPECIFICATION

(45) Date of publication of patent specification: **22.07.87**

(51) Int. Cl.[4]: **C 07 D 403/04,** A 61 K 31/415

(21) Application number: **84300649.5**

(22) Date of filing: **02.02.84**

(54) **(5-(2-Aminomethyl-imidazol-1-yl)-1,3-dialkyl-1H-pyrazol-4-yl)(aryl)methanones and processes for their production.**

(30) Priority: **04.02.83 US 463795**

(43) Date of publication of application:
**12.09.84 Bulletin 84/37**

(45) Publication of the grant of the patent:
**22.07.87 Bulletin 87/30**

(84) Designated Contracting States:
**AT BE CH DE FR GB IT LI LU NL SE**

(56) References cited:
**GB-A-1 484 968**

(73) Proprietor: **WARNER-LAMBERT COMPANY**
**201 Tabor Road**
**Morris Plains New Jersey 07950 (US)**

(72) Inventor: **Butler, Donald Eugene**
**1449 Covington Drive**
**Ann Arbor Michigan 48103 (US)**
Inventor: **De Wald, Horace Albert**
**1265 Bardstown Trail**
**Ann Arbor Michigan 48105 (US)**

(74) Representative: **Jones, Michael Raymond et al**
**HASELTINE LAKE & CO. Hazlitt House**
**28 Southampton Buildings Chancery Lane**
**London WC2A 1AT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 118 205 B1

Courier Press, Leamington Spa, England.

**Description**

U.S. Patent 3,915,981 describes the compound of the formula

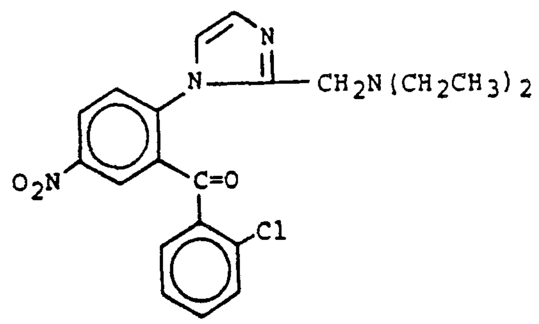

which is said to possess protective activities on cerebral hypoxic or anoxic conditions and is used for the treatment of cerebral dysfunction, e.g., cerebral arteriosclerosis, cerebral apoplexy, and head trauma.

U.S. Patent 3,763,179 describes a broad class of compounds similar to the above-mentioned patent as sedatives and tranquilisers. The generic structural formula is

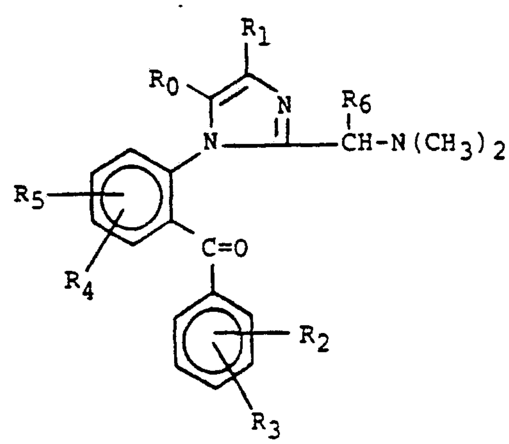

Finally, U.S. Patent 3,927,011 describes compounds of the generic formula

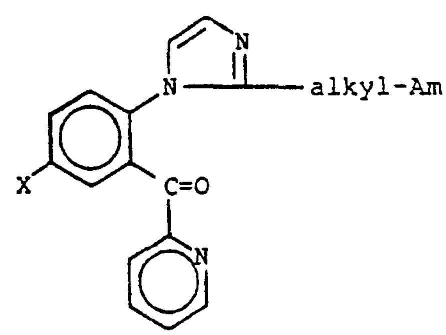

having the same biological activity as in U.S. Patent 3,915,981.

The compounds of the present invention, described below, are cognition activators and are useful in treating senility and induced amnesia.

The present invention relates to compounds of the formula

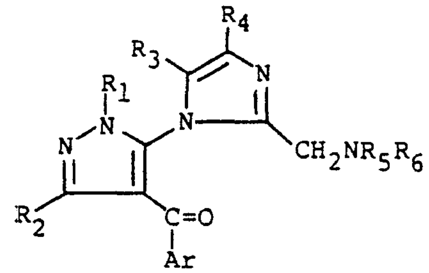

I

and pharmaceutically acceptable acid-addition salts thereof, and to a method for the production of the foregoing compounds; where $R_1$ and $R_2$ are alkyl having one to six carbon atoms, $R_3$ and $R_4$ are hydrogen or alkyl having one to six carbon atoms, $R_5$ and $R_6$ are hydrogen, alkyl having one to six carbon atoms, phenyl lower, $C_{1-6}$ alkyl, or phenyl lower, $C_{1-6}$, alkyl substituted by halo, alkyl having one to four carbon atoms, alkoxy having one to four carbon atoms or trifluoromethyl, or when taken together form a 5- or 6-membered saturated ring with the nitrogen atom and may be further interrupted in the ring by an additional nitrogen or oxygen atom and which 5- or 6-membered ring may be substituted by alkyl having one to six carbon atoms, and Ar is phenyl, phenyl substituted by halo, alkyl having one to four carbon atoms, alkoxy having one to four carbon atoms, or trifluoromethyl; 2- or 3-thienyl or 2- or 3-thienyl substituted by alkyl having one to four carbon atoms.

The terms "alkyl" and "lower alkyl," unless otherwise indicated, are intended to mean a straight or branched hydrocarbon chain having at most six carbon atoms.

When $R_5$ and $R_6$ are taken together, they form a 5- or 6-membered ring with the nitrogen atom resulting in the pyrrolidine or piperidino group which groups may be further substituted by alkyl having one to four carbon atoms such as, for example, methyl and ethyl. The 5- or 6-membered ring further may be interrupted by an additional heteroatom such as nitrogen or oxygen to form groups such as piperazino or morpholino which groups may also be substituted by alkyl having one to four carbon atoms such as, for example, methyl or ethyl.

The free bases of the invention form acid-addition salts with any of a variety of organic and inorganic acids. Pharmaceutically acceptable acid-addition salts are formed with such acids as hydrochloric, sulfuric, nitric, phosphoric, acetic, citric, tartaric, succinic, salicylic, maleic, malic, lactic, gluconic, and pamoic acids. In most cases, salts with one equivalent of a mineral acid or a strong organic acid are stable chemical derivatives. The free bases and their salt forms are interconvertible by adjustment of the pH. The free bases are produced by basification and the acid-addition salts are produced by acidification. They differ in solubility properties but, in general, are otherwise equivalent for the purposes of the invention.

Preferred embodiments of the invention are the compounds of the formula I and pharmaceutically acceptable acid addition salts, where $R_1$ and $R_2$ are methyl or ethyl, $R_2$ and $R_3$ are hydrogen, methyl, or ethyl, $R_5$ and $R_6$ are hydrogen, methyl, ethyl or when taken together with nitrogen form an unsubstituted or methyl substituted pyrrolidino, piperidino, piperazino or morpholino group and Ar is phenyl, phenyl substituted by chloro or fluoro, or 2- or 3-thienyl.

Other preferred embodiments are the following compounds and pharmaceutically acceptable acid-addition salts.

[5-((2-aminomethyl)-5-methyl-1*H*-imidazol-1-yl)-1,3-dimethyl-1*H*-pyrazol-4-yl](2-chlorophenyl)methanone dihydrochloride (Example 1);

[5-((2-dimethylaminomethyl)-5-methyl-1*H*-imidazol-1-yl)-1,3-dimethyl-1*H*-pyrazol-4-yl](2-chlorophenyl)methanone (Example 2);

(2-chlorophenyl)-[1,3-dimethyl-5[2-(1-pyrrolidinylmethyl)-1*H*-imidazol-1-yl]-1*H*-pyrazol-4-yl]methanone dihydrochloride (Example 3), and (2-chlorophenyl)-[1,3-dimethyl-5-[4,5-dimethyl-2-[4-methyl-piperazin-1-yl)methyl]-1*H*-imidazol-1-yl])-1*H*-pyrazol-4-yl]methanone (Example 4).

In accordance with the present invention, the foregoing compounds of formula I, wherein $R_5$ and $R_6$ are hydrogen, can be prepared by hydrolyzing a compound of the formula

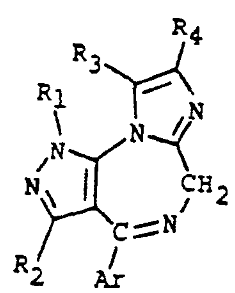

wherein $R_1$, $R_2$, $R_3$, $R_4$ and Ar have the same definitions as in formula I, and when $R_5$ is other than hydrogen,

reacting a compound of the formula II with an excess amount of an aldehyde of the formula $R_5CHO$ in the presence of formic acid.

The hydrolysis of the compounds of the formula II is carried out under aqueous acid conditions at room or elevated temperatures. Preferred conditions involve heating at the boiling point of the mixture with concentrated acid, such as, hydrochloric acid.

The reductive alkylation reaction using an excess of aldehyde in the presence of formic acid is preferably carried out at elevated temperatures, such as from 50—150°C or preferably from 80—120°C.

The compounds of the formula II can be prepared as described in U.S. Patents 4,075,408 and 4,105,851.

In accordance with the present invention, the compounds of the formula I can be prepared by displacing the hydroxyl group on a compound of the formula

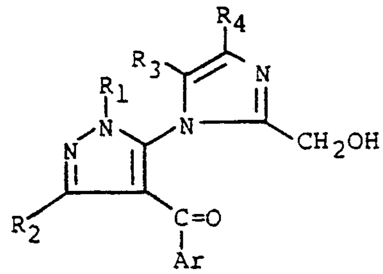

III

with an amine of the formula $R_5R_6NH$ wherein $R_1$, $R_2$, $R_3$, $R_4$, $R_5$, $R_6$, and Ar have been previously defined, said displacement being achieved by converting the hydroxyl group to an ester that is readily displaceable by a nucleophile and reacting the ester with an excess of the appropriate amine of the formula $R_5R_6NH$.

The above displacement reaction is first carried out by activating the hydroxyl group on a compound of formula III. Thus, a compound of the formula III is first reacted with an acid halide of an acid which is capable of forming an ester that is readily displaced by a nucleophile. For example, a compound of the formula III is first treated with p-toluenesulfonyl chloride or methanesulfonyl chloride in the presence of an acid acceptor, such as an organic base, for example, triethylamine. The resulting activated ester is reacted with an excess of the appropriate amine of the formula $R_5R_6NH$ in an inert organic solvent at room temperature or just below room temperature, for example, from 0—30°C or preferably 15—25°C. Organic solvents are those that are inert but polar enough to dissolve the starting materials such as, for example, tetrahydrofuran.

The compounds of the formula III are in turn prepared by first reacting a compound of the formula

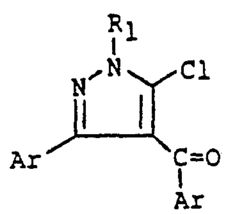

IV

wherein $R_2$, $R_1$ and Ar have been defined above, with an imidazole of the formula

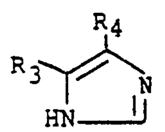

V

wherein $R_3$ and $R_4$ have been defined above, and then treating the resulting product with aqueous formaldehyde.

The first step is carried out using an equimolar amount of sodium hydride at room or below room temperature then warmed slowly to elevated temperature of approximately 100°C. The reaction is carried out in an organic aprotic solvent such as, dimethylformamide, N,N-dimethylacetamide and the like.

The second step is conveniently carried out in formalin (37% aqueous formaldehyde) at the boiling point of the reaction mixture.

The starting materials of the formula IV are prepared as described in J. Org. Chem. *36*, 2542 (1971).

The compounds of this invention are new chemical compounds of value as pharmacological agents. More specifically, they are cognition activators which are potentially useful in treating patients suffering from senility. The compounds also find use in the treatment of induced amnesia.

The compounds of this invention may be administered orally in the form of tablets, capsules, syrups, etc. or parenterally by being dissolved in an appropriate isotonic solution.

The effectiveness of the aforementioned compounds is determined by the test given below which is designed to show the compound's ability to reverse amnesia produced by an electroconvulsive shock.

One hundred male mice (Carworth, CF-1 strain, 19-21 g at time of shipment) are divided into five groups of 20 mice each. Each mouse is placed, one at a time, on a small shelf attached to the outside wall of a text box. In this position the mouse is suspended in space. Therefore, the mouse is motivated to step from the shelf through a conveniently placed small hole into the interior of the box. As soon as the mouse has all four feet within the semidarkened interior of the box, the grid floor of the box is electrified (1.5 milliamps, 3 second duration) to produce a strong pain-fear reaction from the animal. About five seconds thereafter, the mouse is removed from the test box and placed in a group holding cage.

Two hours after the above training, the mice are given a single electroconvulsive shock produced by 20 milliamps delivered for 1.0 seconds through the ears. Immediately thereafter, the mice are returned to the holding cage.

Two hours after the convulsive treatment, the mice are administered orally through a trachea tube with the chemical being assessed. Usually three doses of the chemical will be tested at a time.

One hour after the drug treatment, the mice are tested for memory of the painful foot shock received within the shelf-box apparatus. This testing is accomplished by once again placing each mouse on the small shelf attached to the text box. Any mouse that stays on the shelf for 60 seconds without entering the box is counted as remembering the painful foot shock received within the box five hours earlier. Any mouse entering the box within the 60-second period is counted as having amnesia for the painful event.

Using this 60-second criterion, appropriate control experiments show: 1) 100 percent of mice will enter the box if no foot shock is delivered during the original training, (painful foot shock is necessary if the mice are to develop an aversion to entering the test box) 2) 100 percent of mice will enter the box under the foregoing conditions even when treated with electroconvulsive shock at the three-hour point prior to testing (electroconvulsive shock) treatment itself does not generate a fear of entering the test box).

The five groups of mice are treated as follows:

| Group | Treatments |
|---|---|
| 1. Ceiling Control Group: | Placebo |
| 2. Base Line Control Group: | Electroconvulsive shock, Placebo |
| 3. 1st Drug Dose Group: | Electroconvulsive shock, 5-aminomethyl-imidazolyl-1-yl-1,3-dialkyl-1*H*-pyrazol-4-yl aroyl methanone |
| 4. 2nd Drug Dose Group: | Electronvulsive shock, 5-aminomethyl-imidazolyl-1-yl-1,3-dialkyl-1*H*-pyrazol-4-yl aroyl methanone |
| 5. 3rd Drug Dose Group: | Electroconvulsive shock, 5-aminomethyl-imidazolyl-1-yl-1,3-dialkyl-1*H*-pyrazol-4-yl aroyl methanone |

The percentage of amnesia reversal is determined as follows for each drug group:

$$\text{percent amnesia reversal} = \frac{\text{Drug group-Base line control group}}{\text{Ceiling control group-base line control group}} \times 100$$

The following criteria is used in interpreting the percent of amnesia reversal scores: 40 percent or more (active = A) 25 to 39 percent (borderline = C) and 0 to 29 percent (inactive = N). The following table reports the percent of amnesia reversal of orally administered [(5-(2-aminomethyl)-5-methyl-1*H*-imidazol-1-yl)-1,3-dimethyl-1*H*-pyrazol-4-yl](2-chlorophenyl)methanone dihydrochloride.

TABLE 1

| Dose mg/kg | 0.63 | 1.25 | 2.50 | 5.00 | 20.00 | 80.00 |
|---|---|---|---|---|---|---|
| % Amnesia Reversal Rating | 20 | 40 | 40 | 62 | 87 | 44 |
| | N | A | A | A | A | A |

Accordingly, the present invention includes the compounds of the formula I in the form of pharmaceutical compositions for treating senility or amnesia comprising an effective amount of a compound of the invention with a pharmaceutically acceptable carrier.

The compounds of the present invention may be employed in a method of treating senility or amnesia by administering, preferably orally, to a mammal, an effective amount of a compound of the formula I in the form of a pharmaceutical composition. The estimated mammalian dosage for a 70 kg subject is from 1 to 1500 mg/day (0.014 mg to 21.4 mg/kg of weight per day). Preferably 25 to 750 mg/day (0.36 mg to 10.7 mg/kg of weight per day) optionally in divided portions.

Table 2 reports results obtained over a different range of doses than in Table 1 on further examples of the invention.

TABLE 2

Percent Amnesia Reversal (Rating)

| Example Number | Dose mg/kg 1 | 10 | 100 |
|---|---|---|---|
| 2 | 64(A) | 64(A) | 85(A) |
| 3 | 79(A) | 27(C) | 25(C) |
| 4 | 36(C) | 91(A) | 54(A) |

The invention is further illustrated by the following examples:

Example 1

Synthesis of [5-(2-(aminomethyl)-5-methyl-11H-imidazol-1-yl)-1,3-dimethyl-1H-pyrazol-4-yl] (2-chlorophenyl)methanone dihydrochloride

A solution of 15.0 grams of 4-(2-chlorophenyl)-1,6-dihydro-1,3,9-trimethylimidazo[1,2-a]pyrazolo[4,3-f][1,4]diazepine (U.S. patents 4,075,408 and 4,105,851) is dissolved in 100 ml of concentrated hydrochloric acid and the solution is refluxed 22 hours. The solution is concentrated at reduced pressure and the resulting glass is dissolved in 100 ml of hot isopropanol. The solution is concentrated again and the resulting solid is recrystallized from anhydrous ethanol. The crystals are isolated by filtration, washed with anhydrous diethyl ether and dried in vacuo to yield the title compound. The compound melts at 218—219°C with decomposition.

Example 2

Synthesis of [5-(2-(dimethylaminomethyl)-5-methyl-1H-imidazol-1-yl)1,3-dimethyl-1H-pyrazol-4-yl] (2-chlorophenyl) methanone

A solution of 5.0 grams of 4-(2-chlorophenyl)-1,6-dihydro-1,3,9-trimethylimidazo(1,2-a)pyrazolo[4,3-f][1,4]diazepine is dissolved in four grams of 37% formaldehyde solution and five grams of formic acid is added and the mixture is heated at 100°C for 16 hours. The solution is concentrated at reduced pressure, dissolved in water and neutralized with excess 50% sodium hydroxide solution. The product is extracted with methylene chloride and the organic solution dried over anhydrous magnesium sulfate. The mixture is filtered, concentrated, and triturated with anhydrous diethyl ether. The product is isolated by filtration and dried in vacuo at 100°C. The title compound has a melting point 145—146.5°C.

Example 3

(2-chlorophenyl)-[1,3-dimethyl-5-[2-(1-pyrrolidinylmethyl)-1*H*-imidazol-1-yl]-1*H*-pyrazol-4-yl]methanone dihydrochloride

A suspension of 3.3 g (0.01 mol) of (2-chlorophenyl)[1,3-dimethyl-5-(2-hydroxymethyl-1*H*-imidazol-1-yl)1*H*-pyrazol-4-yl]methanone (C) in 50 ml of methylene chloride is cooled to −5°C and 1.4 g of methanesulfonyl chloride is added. Triethylamine (2 ml) is added dropwise to the stirred suspension to give a solution after stirring overnight at 20°C. The solution is washed with a saturated solution of $NaHCO_3$, dried over anhydrous $MgSO_4$ and evaporated in vacuo to give 3.7 g of the mesyl ester of the starting material.

The mesyl ester 3.7 g (0.01 mol) is dissolved in 50 ml of tetrahydrofuran and 2.5 g (0.035 mol) of pyrrolidine is added. After standing at 20°C for 20 hours, the mixture is evaporated in vacuo. The residue is redissolved in methylene chloride; the solution is washed with sodium bicarbonate solution, dried over anhydrous $MgSO_4$ and evaporated. The residual oil is dissolved in 10 ml of tetrahydrofuran, treated with 3 ml of 20% isopropanolic hydrogen chloride, and diluted with ethyl acetate to induce crystallization. The product is isolated by filtration as the dihydrochloride salt with a melting point of 190—192°C.

Starting Material

The starting material (2-chlorophenyl)[5-(1*H*-imidazol-1-yl)-1*H*-pyrazol-4-yl]methanone (B) is obtained as follows:

A solution of 54 g (0.2 mol) of (5-chloro-1,3-dimethyl-1*H*-pyrazol-4-yl) (2-chlorophenyl) methanone (A) [J. Org. Chem. *36* 2542 (1971)] and 18 g (0.24 mol) of imidazole in 300 ml of dimethylformamide is treated portionwise with 10 g (0.2 mol) of sodium hydride (50% dispersion in oil) at 15—30°C under nitrogen. The mixture is warmed slowly to 100°C with stirring and maintained at this temperature for three hours and then evaporated in vacuo. The residue is dissolved in a mixture of 300 ml of methylene chloride and 150 ml of water. The organic layer is separated and washed (extracted) with 1N sodium hydroxide. The organic solution is dried over anhydrous $MgSO_4$ and concentrated and diluted with petroleum ether to give 55 g of (2-chlorophenyl)[5-(1*H*-imidazol-1-yl)-1,3-dimethyl-1*H*-pyrazol-4-yl]methanone (B) mp 93—95°C.

The starting material (2-chlorophenyl)[1,3-dimethyl-5-[2-hydroxymethyl-1*H*-imidazol-1-yl]-1*H*-pyrazol-4-yl]methanone (C) is obtained as follows:

A mixture of 30 g (0.2 mol) of B and 100 g of 37% formalin is stirred and refluxed for 52 hours, and evaporated in vacuo. The oil is dissolved in hot toluene; the toluene solution is washed with hot water, then is extracted with 250 ml of 1N hydrochloric acid. The acid extract is made basic with concentrated ammonium hydroxide. The precipitated product is collected by filtration to give 15.6 g of (2-chlorophenyl)[1,3-dimethyl-5-[2-hydroxymethyl-1*H*-imidazol-1-yl]-1*H*-pyrazol-4-yl]methanone with a melting point of 183—185°C.

## Example 4

(2-Chlorophenyl)[1,3-dimethyl-5-[4,5-dimethyl-2-[(4-methyl-piperazin-1-yl)methyl]-1H-imidazol-1-yl]-1*H*-pyrazol-4-yl]methanone

By the same procedure described in Example 3 a solution of 3.6 g (0.01 mol) intermediate *C'* ($R_1R_2R_3R_4$ = $CH_3$; Ar = 2-chlorophenyl) in 50 ml methylene dichloride is treated with 1.4 g of methanesulfonyl chloride in the presence of triethylamine. The resulting mesyl ester is treated with 3 g N-methylpiperazine in tetrahydrofuran for 20 hours at 20°C. The crude product is purified by passage through neutral alumina and isolated as the dihydrochloride salt (3 g). The crude hydrochloride salt is recrystallized from chloroform-ether to give 1.6 g (33%) mp 175—180°C.

The starting material (2-chlorophenyl)[1,3-dimethyl-5-(4,5-dimethyl-1*H*-imidazol-1-yl)-1*H*-pyrazol-4-yl methanone is obtained as follows:

A solution of 54 g (0.2 mol) of (5-chloro-1,3-dimethyl-1*H*-pyrazol-4-yl) (2-chlorophenyl) methanone (A) and 20 g (0.2 mol) of 4,5-dimethylimidazole in 250 ml of dimethylformamide is treated with 10 g (0.2 mol) of sodium hydride (50% dispersion in oil) and stirred at 90—100°C for four hours to give 46 g of (2-chlorophenyl) [1,3-dimethyl-5-(4,5-dimethyl-1*H*-imidazol-1-yl)-1*H*-pyrazol-4-yl]methanone, mp 141—143°C from ethyl acetate — petroleum ether (B') ($R_1R_2R_3R_4$ = $CH_3$, Ar = 2 chlorophenyl).

Intermediate (2-chlorophenyl)[1,3-dimethyl-5-[4,5-dimethyl-2-hydroxymethyl-1*H*-imidazol-1-yl)-1*H*-pyrazol-4-yl]methanone (B') is obtained as follows:

1,3-Dimethyl-5-(4,5-dimethyl-1*H*-imidazol-1-yl)-1*H*-pyrazol-4-yl]methanone twenty grams, 0.06 mol is heated at 105°C for 18 hours in 60 ml of 37% formalin to yield 11 g (51%) of (2-chlorophenyl) [1,3-dimethyl-5-(4,5-dimethyl-2-hydroxymethyl-1*H*-imidazol-1-yl)-1*H*-pyrazol-4-yl]methanone (B'), with a melting point of 156—159°C after recrystallization from diethyl ether.

For the sake of clarity, all the radicals "R", labelled in the description with a subscript, have been labelled in the claims with the corresponding superscript.

**Claims for the Contracting States: BE CH DE FR GB IT LI LU NL SE**

1. A compound having the following general formula I:

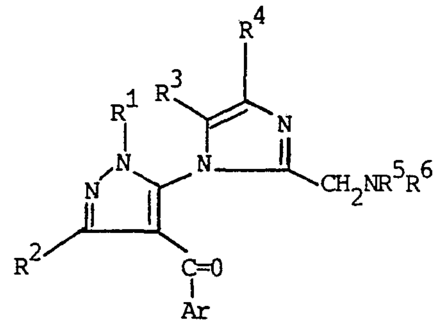

or a pharmaceutically acceptable acid-addition salt thereof, wherein;

$R^1$ and $R^2$, which may be the same or different, are alkyl radicals having from one to six carbon atoms;

$R^3$ and $R^4$, which may be the same or different, are hydrogen atoms or alkyl radicals, having from one to six carbon atoms;

$R^5$ and $R^6$, which may be the same or different, are hydrogen atoms, alkyl radicals having from one to six carbon atoms, phenyl lower, $C_{1-6}$, alkyl radicals, phenyl lower, $C_{1-6}$, alkyl radicals substituted by a halo atom, an alkyl radical having from one to four carbon atoms, an alkoxy having from one to four carbon atoms, or a trifluoromethyl radical; or, when taken together, $R^5$ and $R^6$ form, with the N atom to which they are attached, a 5- or 6-membered saturated ring which may contain a further nitrogen atom or an oxygen atom, and which ring may be singly or multiply substituted by an alkyl radical having from one to six carbon atoms; and

Ar is a phenyl radical; a phenyl radical substituted by a halo atom, an alkyl radical having from one to four carbon atoms, an alkoxy radical having from one to four carbon atoms, or a trifluoromethyl radical; a 2- or 3-thienyl radical; or a 2- or 3-thienyl radical substituted by an alkyl radical having from one to four carbon atoms.

2. A compound according to Claim 1, wherein:

each of $R^1$ and $R^2$ is a methyl radical or an ethyl radical;

each of $R^2$ and $R^3$ is a hydrogen atom, a methyl radical, or an ethyl radical;

each of $R^5$ and $R^6$ is a hydrogen atom, a methyl radical, or an ethyl radical; or $R_5$ and $R_6$, when taken together and with the nitrogen atom to which they are attached, form an unsubstituted or methyl-substituted pyrrolidino, piperadino, piperazino, or morpholino group; and

Ar is a phenyl radical substituted by a chloro or fluoro atom; or a 2- or 3-thienyl radical.

3. A compound according to Claim 2, which is [5-(2-(aminomethyl)-5-methyl-1*H*-imidazol-1-yl)-1,3-dimethyl-1*H*-pyrazol-4-yl](2-chlorophenyl)methanone dihydrochloride.

4. A compound according to Claim 2, which is (2-chlorophenyl)-[1,3-dimethyl-5[2-(1-pyrrolidinylmethyl)-1*H*-imidazol-1-yl]-1*H*-pyrazol-4-yl]methanone dihydrochloride.

5. A compound according to Claim 2, which is [5-(2-(dimethylaminomethyl)-5-methyl-1*H*-imidazol-1-yl)-1,3-dimethyl-1*H*-pyrazol-4-yl](2-chlorophenyl)-methanone.

6. A compound according to Claim 2, which is (2-chlorophenyl)-[1,3-dimethyl-5-[4,5-dimethyl-2-[(4-methyl-piperazin-1-yl)-methyl]-1*H*-imidazol-1-yl]-1*H*-pyrazol-4-yl]methanone.

7. A process for preparing a compound of the general formula I as defined in Claim 1, wherein $R^5$ and $R^6$ are hydrogen atoms, which process comprises hydrolyzing, under aqueous acid conditions, a compound of the formula

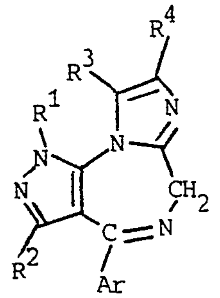

wherein $R^1$, $R^2$, $R^3$, $R^4$ and Ar are as defined in Claim 1.

8. A process for preparing a compound of the general formula I as defined in Claim 1, wherein $R^6$ is a hydrogen atom and $R^5$ is other than a hydrogen atom, which process comprises reacting a compound of the formula

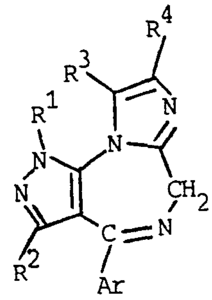

with an excess of an aldehyde of the formula $R^5CHO$ in the presence of formic acid, wherein $R^1$, $R^2$, $R^3$, $R^4$, $R^5$ and Ar are as defined in Claim 1.

9. A process for preparing a compound of the general formula I as defined in Claim 1, which process comprises displacing the hydroxyl group on a compound of the formula

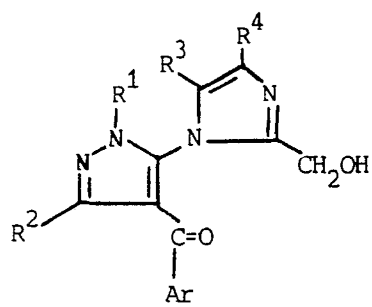

with an amine of the formula $R^5R^6NH$ wherein $R^1$, $R^2$, $R^3$, $R^4$, $R^5$, $R^6$, and Ar are as defined in Claim 1, said displacement being achieved by converting the hydroxyl group to an ester that is readily displaceable by a nucleophile and reacting the ester with an excess of the appropriate amine of formula $R^5R^6NH$.

10. A pharmaceutical composition for treating senility or amnesia, comprising an effective amount of a compound as claimed in any one of Claims 1 to 6, and a pharmaceutically acceptable carrier.

11. For use in a method of treating senility or amnesia, a compound as claimed in any one of Claims 1 to 6 or a pharmaceutical composition as claimed in Claim 10.

**Claims for the Contracting State: AT**

1. A process for preparing a compound having the following general formula I:

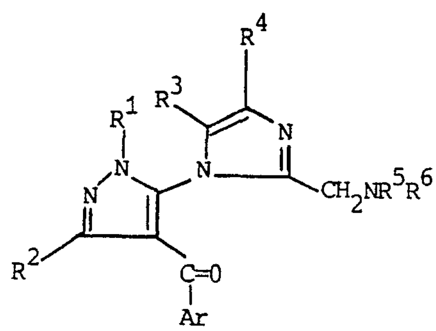

or a pharmaceutically acceptable acid-addition salt thereof, wherein;

$R^1$ and $R^2$, which may be the same or different, are alkyl radicals having from one to six carbon atoms;

$R^3$ and $R^4$, which may be the same or different, are hydrogen atoms or alkyl radicals, having from one to six carbon atoms;

$R^5$ and $R^6$, which may be the same or different, are hydrogen atoms, alkyl radicals having from one to six carbon atoms, phenyl lower, $C_{1-6}$, alkyl radicals, phenyl lower, $C_{1-6}$, alkyl radicals substituted by a halo atom, an alkyl radical having from one to four carbon atoms, an alkoxy having from one to four carbon atoms, or a trifluoromethyl radical; or, when taken together, $R^5$ and $R^6$ form, with the N atom to which they are attached, a 5- or 6-membered saturated ring which may contain a further nitrogen atom or an oxygen atom, and which ring may be singly or multiply substituted by an alkyl radical having from one to six carbon atoms; and

Ar is a phenyl radical; a phenyl radical substituted by a halo atom, an alkyl radical having from one to four carbon atoms, an alkoxy radical having from one to four carbon atoms or a trifluoromethyl radical; a 2- or 3-thienyl radical; or a 2- or 3-thienyl radical substituted by an alkyl radical having from one to four carbon atoms, which process comprises displacing the hydroxyl group on a compound of the formula

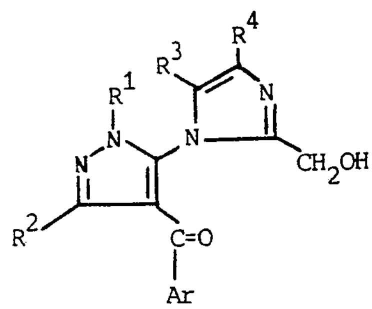

with an amine of the formula $R^5R^6NH$ wherein $R^1$, $R^2$, $R^3$, $R^4$, $R^5$, $R^6$, and Ar are as defined above, said displacement being achieved by converting the hydroxyl group to an ester that is readily displaceable by a nucleophile and reacting the ester with an excess of the appropriate amine of the formula $R^5R^6NH$.

2. A process for preparing a compound of the general formula I as defined in Claim 1, wherein $R^5$ and $R^6$ are hydrogen atoms, which process comprises hydrolyzing, under aqueous acid conditions, a compound of the formula

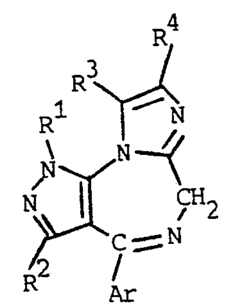

wherein $R^1$, $R^2$ $R^3$, $R^4$ and Ar are as defined in Claim 1.

3. A process for preparing a compound of the general formula I as defined in Claim 1, wherein $R^6$ is a hydrogen atom and $R^5$ is other than a hydrogen atom, which process comprises reacting a compound of the formula

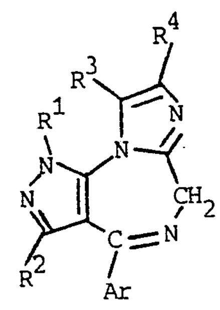

with an excess of an aldehyde of the formula $R^5CHO$ in the presence of formic acid, wherein $R^1$, $R^2$, $R^3$, $R^4$, $R^5$ and Ar are as defined in Claim 1.

4. A process according to Claim 1, 2 or 3, wherein:

each of $R^1$ and $R^2$ is a methyl radical or an ethyl radical;

each of $R^2$ and $R^3$ is a hydrogen atom, a methyl radical, or an ethyl radical;

each of $R^5$ and $R^6$ is a hydrogen atom, a methyl radical, or an ethyl radical; or $R_5$ and $R_6$, when taken together and with the nitrogen atom to which they are attached, form an unsubstituted or methyl-substituted pyrrolidino, piperadino, piperazino, or morpholino group; and

Ar is a phenyl radical substituted by a chloro or fluoro atom; or a 2- or 3-thienyl radical.

5. A process according to Claim 1 or 2, for preparing the compound [5-(2-(aminomethyl)-5-methyl-1*H*-imidazol-1-yl)-1,3-dimethyl-1*H*-pyrazol-4-yl](2-chlorophenyl)methanone dihydrochloride.

6. A process according to Claim 1, for preparing the compound (2-chlorophenyl)-[1,3-dimethyl-5[2-(1-pyrrolidinylmethyl)-1*H*-imidazol-1-yl-1*H*-pyrazol-4-yl]methanone dihydrochloride.

7. A process according to Claim 1, for preparing the compound [5-(2-(dimethylaminomethyl)-5-methyl-1*H*-imidazol-1-yl)-1,3-dimethyl-1*H*-pyrazol-4-yl](2-chlorophenyl)-methanone.

8. A process according to Claim 1, for preparing the compound (2-chlorophenyl)-[1,3-dimethyl-5-[4,5-dimethyl-2-[(4-methyl-piperazin-1-yl)-methyl]-1*H*-imidazol-1-yl]-1*H*-pyrazol-4-yl]methanone.

9. A process for preparing a pharmaceutical composition for treating senility or amnesia, comprising combining an effective amount of a compound prepared in accordance with any one of Claims 1 to 8 with a pharmaceutically acceptable carrier.

10. For use in a method of treating senility or amnesia, a compound prepared by a process as claimed in any preceding claim or a pharmaceutical composition prepared by a process as claimed in Claim 9.

**Patentansprüche für die Vertragsstaaten: BE CH DE FR GB IT LI LU NL SE**

1. Verbindung mit der folgenden allgemeinen Formel I

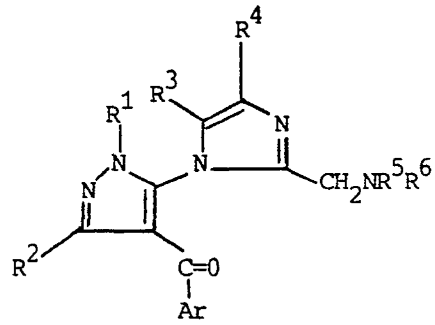

oder ein pharmazeutisch akzeptables Säureadditionssalz davon, worin:

$R^1$ und $R^2$, welche gleich oder verschieden sein können, Alkylreste mit 1 bis 6 Kohlenstoffatomen darstellen;

$R^3$ und $R^4$, welche gleich oder verschieden sein können, Wasserstoffatome oder Alkylreste mit 1 bis 6 Kohlenstoffatomen repräsentieren;

$R^5$ und $R^6$, welche gleich oder verschieden sein können, Wasserstoffatome, Alkylradikale mit 1 bis 6 Kohlenstoffatomen, Phenyl-niedrig$C_{1-6}$alkylradikale, durch ein Halogenatom, ein Alkylradikal mit 1 bis 4 Kohlenstoffatomen, ein alkoxyradikal mit 1 bis 4 Kohlenstoffatomen oder ein Trifluormethylradikal substituierte Phenylniedrig$C_{1-6}$alkylradikale darstellen; oder zusammengenommen $R^5$ und $R^6$ mit dem Stickstoffatom, an welches sie gebunden sind, einen 5- oder 6-gliedrigen gesättigten Ring bilden, welcher ein weiteres Stickstoffatom oder ein Sauerstoffatom enthalten kann, und welcher Ring einfach oder mehrfach durch einen Alkylrest mit 1 bis 6 Kohlenstoffatomen substituiert sein kann; und Ar für ein Phenylradikal, ein durch ein Halogenatom, ein Alkylradikal mit 1 bis 4 Kohlenstoffatomen, ein Alkoxyradikal mit 1 bis 4 Kohlenstoffatomen oder ein Trifluormethylradikal substituiertes Phenylradikal; ein 2- oder 3-Thienylradikal; oder ein durch einen Alkylrest mit 1 bis 4 Kohlenstoffatomen substituiertes 2- oder 3-Thienylradikal steht.

2. Verbindung nach Anspruch 1, worin:

jedes von $R^1$ und $R^2$ ein Methylradikal oder ein Äthylradikal bedeutet;

jedes von $R^2$ und $R^3$ für ein Wasserstoffatom, ein Methylradikal oder einen Äthylrest steht;

jedes von $R^5$ und $R^6$ ein Wasserstoffatom, ein Methylradikal oder ein Äthylrest ist; oder $R^5$ und $R^6$ zusammengenommen und mit dem Stickstoffatom, an welches sie gebunden sind, eine unsubstituierte oder methylsubstituierte Pyrrolidino-, Piperidino-, Piperazino- oder Morpholinogruppe bilden; und

Ar für ein durch ein Chlor- oder Fluoratom substituiertes Phenylradikal; oder ein 2- oder 3-Thienylradikal steht.

3. Verbindung nach Anspruch 2, welche [5-(2-(Aminomethyl)-5-methyl-1H-imidazol-1-yl)-1,3-dimethyl-1H-pyrazol-4-yl]-(2-chlorphenyl)-methanon-dihydrochlorid ist.

4. Verbindung nach Anspruch 2, welche (2-Chlorphenyl)-[1,3-dimethyl-5-[2-(1-pyrrolidinylmethyl)-1H-imidazol-1-yl]-1H-pyrazol-4-yl]-methanon-dihydrochlorid ist.

5. Verbindung nach Anspruch 2, welche [5-(2-(Dimethylaminomethyl)-5-methyl-1H-imidazol-1-yl)-1,3-dimethyl-1H-pyrazol-4-yl]-(2-chlorphenyl)-methanon ist.

6. Verbindung nach Anspruch 2, welche (2-Chlorphenyl)-[1,3-dimethyl-5-[4,5-dimethyl-2-[(4-methyl-piperazin-1-yl)-methyl]-1H-imidazol-1-yl]-1H-pyrazol-4-yl]-methanon ist.

7. Verfahren zur Herstellung einer wie im Anspruch 1 definierten Verbindung der allgemeinen Formel I, worin $R^5$ und $R^6$ Wasserstoffatome darstellen, welches Verfahren die Hydrolyse einer Verbindung der Formel

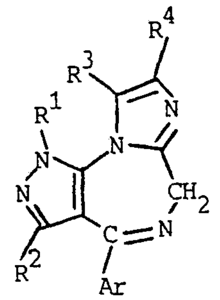

worin $R^1$, $R^2$, $R^3$, $R^4$ und Ar wie im Anspruch 1 definiert sind, unter wässerig-sauren Bedingungen umfaßt.

8. Verfahren zur Herstellung einer wie im Anspruch 1 definierten Verbindung mit der allgemeinen Formel I, worin $R^6$ für ein Wasserstoffatom steht, und $R^5$ eine andere Bedeutung als Wasserstoffatom hat, welches Verfahren die Reaktion einer Verbindung der Formel

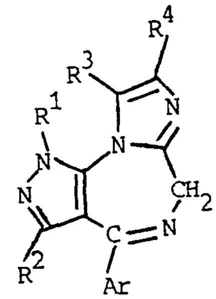

mit einem Überschuß an einem Aldehyd der Formel $R^5CHO$, worin $R^1$, $R^2$, $R^3$, $R^4$, $R^5$ und Ar wie im Anspruch 1 definiert sind, in Gegenwart von Ameisensäure umfaßt.

9. Verfahren zur Herstellung einer wie im Anspruch 1 definierten Verbindung der allgemeinen Formel I, welches Verfahren den Ersatz der Hydroxylgruppe in einer Verbindung der Formel

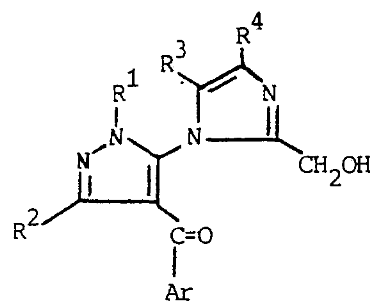

mit einem Amin der Formel $R^5R^6NH$, worin $R^1$, $R^2$, $R^3$, $R^4$, $R^5$, $R^6$ und Ar wie im Anspruch 1 definiert sind, umfaßt, wobei dieser Ersatz durch Überführung der Hydroxylgruppe in einen durch ein Nucleophil leicht ersetzbaren Ester und Umsetzung des Esters mit einem Überschuß des entsprechenden Amins der Formel $R^5R^6NH$ erreicht wird.

10. Pharmazeutische Zusammensetzung zur Behandlung von Senilität oder Amnesie, welche eine wirksame Menge einer Verbindung nach irgendeinem der Ansprüche 1 bis 6 und einen pharmazeutisch akzeptablen Träger umfaßt.

11. Verbindung nach irgendeinem der Ansprüche 1 bis 6 oder eine pharmazeutische Zusammensetzung nach Anspruch 10 zur Verwendung bei einer Methode zur Behandlung von Senilität oder Amnesie.

**Patentansprüche für den Vertragsstaat: AT**

1. Verfahren zur Herstellung einer Verbindung mit der folgenden allgemeinen Formel I:

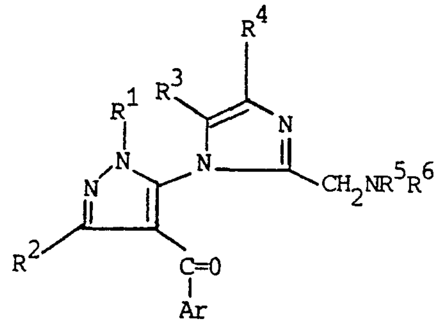

oder eines pharmazeutisch akzeptable Säureadditionssalzes davon, worin:

$R^1$ und $R^2$, welche gleich oder verschieden sein können, Alkylreste mit 1 bis 6 Kohlenstoffatomen darstellen;

$R^3$ und $R^4$, welche gleich oder verschieden sein können, Wasserstoffatome oder Alkylreste mit 1 bis 6 Kohlenstoffatomen sind;

$R^5$ und $R^6$, welche gleich oder verschieden sein können, Wasserstoffatome, Alkylradikale mit 1 bis 6 Kohlenstoffatomen, Phenyl-niedrig$C_{1-6}$alkyl, durch ein Halogenatom, ein Alkylradikal mit 1 bis 4 Kohlenstoffatomen, ein alkoxyradikal mit 1 bis 4 Kohlenstoffatomen oder ein Trifluormethylradikal substituierte Phenylniedrig$C_{1-6}$alkylradikale darstellen; oder zusammengenommen $R^5$ und $R^6$ mit dem Stickstoffatom, an welches sie gebunden sind, einen 5- oder 6-gliedrigen gesättigten Ring bilden, welcher ein weiteres Stickstoffatom oder ein Sauerstoffatom enthalten kann, und welcher Ring einfach oder mehrfach durch einen Alkylrest mit 1 bis 6 Kohlenstoffatomen substituiert sein kann; und Ar für ein Phenylradikal, ein durch ein Halogenatom, ein Alkylradikal mit 1 bis 4 Kohlenstoffatomen, ein Alkoxyradikal mit 1 bis 4 Kohlenstoffatomen oder ein Trifluormethylradikal substituiertes Phenylradikal; ein 2- oder 3-Thienylradikal; oder ein durch ein Alkylradikal mit 1 bis 4 Kohlenstoffatomen substituiertes 2- oder 3-Thienylradikal steht, welches Verfahren den Ersatz der Hydroxylgruppe in einer Verbindung der Formel

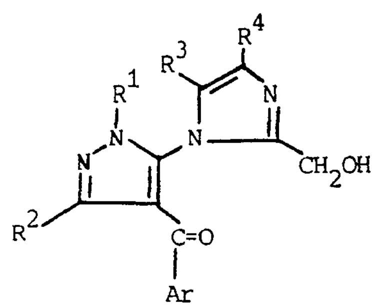

mit einem Amin der Formel $R^5R^6NH$, worin $R^1$, $R^2$, $R^3$, $R^4$, $R^5$, $R^6$ und Ar wie oben definiert sind, umfaßt, wobei der Ersatz durch Überführung der Hydroxylgruppe in einen durch ein Nucleophil leicht ersetzbaren Ester und Umsetzung des Esters mit einem Überschuß des entsprechenden Amins der Formel $R^5R^6NH$ erreicht wird.

2. Verfahren zur Herstellung einer wie im Anspruch 1 definierten Verbindung der allgemeinen Formel I, worin $R^5$ und $R^6$ Wasserstoffatome repräsentieren, welches Verfahren die Hydrolyse einer Verbindung der Formel

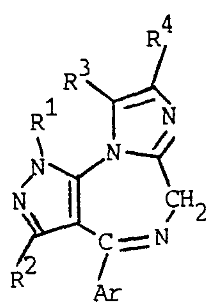

worin $R^1$, $R^2$, $R^3$, $R^4$ und Ar wie im Anspruch 1 definiert sind, unter wässerig-sauren Bedingungen umfaßt.

3. Verfahren zur Herstellung einer wie im Anspruch 1 definierten Verbindung der allgemeinen Formel I, worin $R^6$ für ein Wasserstoffatom steht, und $R^5$ eine andere Bedeutung als Wasserstoffatom hat, welches Verfahren die Reaktion einer Verbindung der Formel

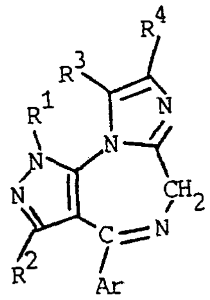

mit einem Überschuß an einem Aldehyd der Formel $R^5CHO$, worin $R^1$, $R^2$, $R^3$, $R^4$, $R^5$ und Ar wie im Anspruch 1 definiert sind, in Gegenwart von Ameisensäure umfaßt.

4. Verfahren nach Anspruch 1, 2 oder 3, worin:

jedes von $R^1$ und $R^2$ ein Methylradikal oder ein Äthylradikal bedeutet;

jedes von $R^2$ und $R^3$ für ein Wasserstoffatom, ein Methylradikal oder einen Äthylrest steht;

jedes von $R^5$ und $R^6$ ein Wasserstoffatom, ein Methylradikal oder ein Äthylrest ist; oder $R^5$ und $R^6$ zusammengenommen und mit dem Stickstoffatom, an welches sie gebunden sind, eine unsubstituierte oder methylsubstituierte Pyrrolidino-, Piperidino-, Piperazino- oder Morpholinogruppe bilden; und

Ar für ein durch ein Chlor- oder Fluoratom substituiertes Phenylradikal; oder ein 2- oder 3-Thienylradikal steht.

5. Verfahren nach Anspruch 1 oder 2 zur Herstellung der Verbindung [5-(2-(Aminomethyl)-5-methyl-1H-imidazol-1-yl)-1,3-dimethyl-1H-pyrazol-4-yl]-(2-chlorphenyl)-methanon-dihydrochlorid.

6. Verfahren nach Anspruch 1 zur Herstellung der Verbindung (2-Chlorphenyl)-[1,3-dimethyl-5-[2-(1-pyrrolidinylmethyl)-1H-imidazol-1-yl]-1H-pyrazol-4-yl]-methanondihydrochlorid.

7. Verfahren nach Anspruch 1 zur Herstellung der Verbindung [5-(2-Dimethylaminomethyl)-5-methyl-1H-imidazol-1-yl)-1,3-dimethyl-1H-pyrazol-4-yl]-(2-chlorphenyl)-methanon.

8. Verfahren nach Anspruch 1 zur Herstellung der Verbindung (2-Chlorphenyl)-[1,3-dimethyl-5-[4,5-dimethyl-2-[(4-methyl-piperazin-1-yl)-methyl]-1H-imidazol-1-yl]-1H-pyrazol-4-yl]-methanon.

9. Verfahren zur Herstellung einer pharmazeutischen Zusammensetzung zur Behandlung von Senilität oder Amnesie, welches die Kombination einer wirksamen Menge einer in Übereinstimmung mit irgendeinem der Ansprüche 1 bis 8 hergestellten Verbindung mit einem pharmazeutisch akzeptablen Träger umfaßt.

10. Verbindung, welche mittels eines Verfahrens nach irgendeinem der vorhergehenden Ansprüche hergestellt wird, oder eine mittels eines Verfahrens nach Anspruch 9 hergestellte pharmazeutische Zusammensetzung zur Verwendung bei einer Methode zur Behandlung von Senilität oder Amnesie.

**Revendications pour les Etats contractants: BE CH DE FR GB IT LI LU NL SE**

1. Un composé de formule générale I:

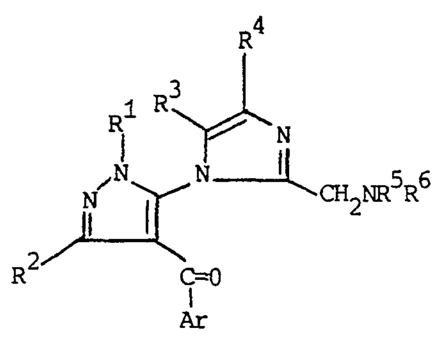

ou un de ses sels d'addition d'acides pharmaceutiquement acceptables, dans laquelle:

$R^1$ et $R^2$, qui peuvent être identiques ou différents, représentent des radicaux alkyles renfermant de 1 à 6 atomes de carbone;

$R^3$ et $R^4$, qui peuvent être identiques ou différents, représentent des atomes d'hydrogène ou des radicaux alkyles renfermant de 1 à 6 atomes de carbone;

$R^5$ et $R^6$, qui peuvent être identiques ou différents, représentent des atomes d'hydrogène, des radicaux alkyles renfermant de 1 à 6 atomes de carbone, des radicaux (alkyl inférieur en $C_1$ à $C_6$)-phényle, des radicaux (alkyl inférieur en $C_1$ à $C_6$)-phényle dans lesquels le radical alkyle est substitué par un atome d'halogène, un radical alkyle renfermant de 1 à 4 atomes de carbone, un radical alkoxy renfermant de 1 à 4 atomes de carbone ou un radical trifluorométhyle ou, lorsqu'ils sont pris ensemble, $R^5$ et $R^6$ forment avec l'atome de N auquel ils sont attachés un cycle saturé à 5 ou 6 éléments qui peut contenir un autre atome d'azote ou un autre atome d'oxygène, et ce cycle pouvant être substitué de façon unique ou multiple par un radical alkyle renfermant de 1 à 6 atomes de carbone; et

Ar représente le radical phényle, un radical phényle substitué par un atome d'halogène, un radical alkyle renfermant de 1 à 4 atomes de carbone, un radical alkoxy renfermant de 1 à 4 atomes de carbone, ou un radical trifluorométhyle; un radical 2- ou 3-thiényle ou un radical 2- ou 3-thiényle substitué par un radical alkyle renfermant de 1 à 4 atomes de carbone.

2. Un composé selon la revendication 1, dans lequel:

$R^1$ et $R^2$ représentent chacun un radical méthyle ou un radical éthyle;

$R^2$ et $R^3$ représentent chacun un atome d'hydrogène, un radical méthyle ou un radical éthyle;

$R^5$ et $R^6$ représentent chacun un atome d'hydrogène, un radical méthyle ou un radical éthyle; ou $R^5$ et $R^6$ pris ensemble et avec l'atome d'azote auquel ils sont attachés, forment un groupe pyrrolidino, pipéridino, pipérazino ou morpholino non substitué ou substitué par un méthyle; et

Ar représente un radical phényle substitué par un atome de chlore ou de fluor; ou un radical 2- ou 3-thiényle.

3. Un composé selon la revendication 2, qui est le dichlorhydrate de {5-[2-(aminométhyl)-5-méthyl-1H-imidazol-1-yl]-1,3-diméthyl-1H-pyrazol-4-yl}(2-chlorophényl)méthanone.

4. Un composé selon la revendication 2, qui est le dichlorhydrate de (2-chlorophényl)-{1,3-diméthyl-5-[2-(1-pyrrolidinylméthyl)-1H-imidazol-1-yl]-1H-pyrazol-4-yl}méthanone.

5. Un composé selon la revendication 2, qui est la {5-[2-(aminométhyl)-5-méthyl-1H-imidazol-1-yl]-1,3-diméthyl-1H-pyrazol-4-yl}(2-chlorophényl)méthanone.

6. Un composé selon la revendication 2, qui est la (2-chlorophényl)-{1,3-diméthyl-5-[4,5-diméthyl-2-[(4-méthyl-pipérazin-1-yl)-méthyl]-1H-imidazol-1-yl]-1H-pyrazol-4-yl}méthanone.

7. Un procédé de préparation d'un composé de formule générale I, tel que défini dans la revendication 1, dans lequel $R^5$ et $R^6$ représentent des atomes d'hydrogène, ce procédé consistant à hydrolyser, dans des conditions acides aqueuses, un composé de formule:

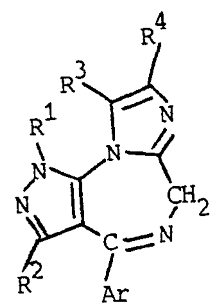

dans laquelle $R^1$, $R^2$, $R^3$, $R^4$ et Ar sont tels que définis dans la revendication 1.

8. Un procédé de préparation d'un composé de formule générale I, tel que défini dans la revendication 1, dans lequel $R^6$ représente un atome d'hydrogène et $R^5$ représente autre chose qu'un atome d'hydrogène, ce procédé consistant à faire réagir un composé de formule:

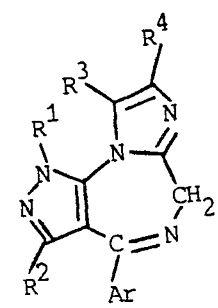

avec un excès d'un aldéhyde de formule $R^5CHO$ en présence d'acide formique, dans laquelle $R^1$, $R^2$, $R^3$, $R^4$, $R^5$ et Ar sont tels que définis dans la revendication 1.

9. Un procédé de préparation d'un composé de formule générale I, tel que défini dans la revendication 1, ce procédé consistant à déplacer le groupe hydroxyle d'un composé de formule:

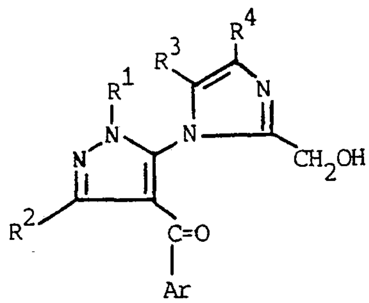

avec une amine de formule $R^5R^6NH$, dans laquelle $R^1$, $R^2$, $R^3$, $R^4$, $R^5$, $R^6$ et Ar sont tels que définis dans la revendication 1, ce déplacement étant obtenu en transformant le groupe hydroxyle en un ester qui se déplace facilement par un nucléophile, et à faire réagir l'ester avec un excès de l'amine appropriée de formule $R^5R^6NH$.

10. Un composition pharmaceutique de traitement de la sénilité ou de l'amnésie comprenant une quantité efficace d'un composé selon l'une quelconque des revendications 1 à 6, et un support pharmaceutiquement acceptable.

11. Pour l'utilisation dans un procédé de traitement de la sénilité ou de l'amnésie, un composé selon l'une quelconque des revendications 1 à 6, ou une composition pharmaceutique selon la revendication 10.

**Revendications pour l'Etat contractant: AT**

1. Un procédé de préparation d'un composé de formule générale I:

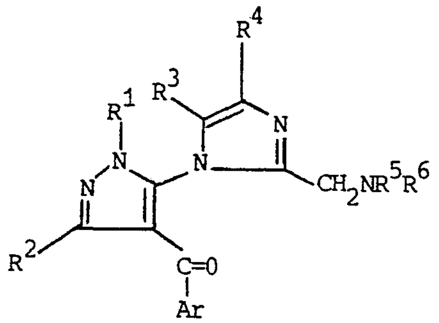

ou d'un de ses sels d'addition d'acides pharmaceutiquement acceptables, dans laquelle:

$R^1$ et $R^2$, qui peuvent être identiques ou différents, représentent des radicaux alkyles renfermant de 1 à 6 atomes de carbone;

$R^3$ et $R^4$, qui peuvent être identiques ou différents, représentent des atomes d'hydrogène ou des radicaux alkyles renfermant de 1 à 6 atomes de carbone;

$R^5$ et $R^6$, qui peuvent être identiques ou différents, représentent des atomes d'hydrogène, des radicaux alkyles renfermant de 1 à 6 atomes de carbone, des radicaux (alkyl inférieur en $C_1$ à $C_6$)-phényle, des radicaux (alkyl inférieur en $C_1$ à $C_6$)-phényle dont le radical alkyle est substitué par un atome d'halogène, un radical alkyle renfermant de 1 à 4 atomes de carbone, un radical alkoxy renfermant de 1 à 4 atomes de carbone ou un radical trifluorométhyle; ou, pris ensemble, $R^5$ et $R^6$ forment avec l'atome de N auquel ils sont attachés un cycle saturé à 5 ou 6 éléments qui peut contenir un autre atome d'azote ou un autre atome d'oxygène, et ce cycle pouvant être substitué de façon unique ou multiple par un radical alkyle renfermant de 1 à 6 atomes de carbone; et

Ar représente le radical phényle, un radical phényle substitué par un atome d'halogène, un radical alkyle renfermant de 1 à 4 atomes de carbone, un radical alkoxy renfermant de 1 à 4 atomes de carbone ou un radical trifluorométhyle; un radical 2- ou 3-thiényle ou un radical 2- ou 3-thiényle substitué par un radical alkyle renfermant de 1 à 4 atomes de carbone, ce procédé consistant à déplacer le groupe hydroxyle d'un composé de formule:

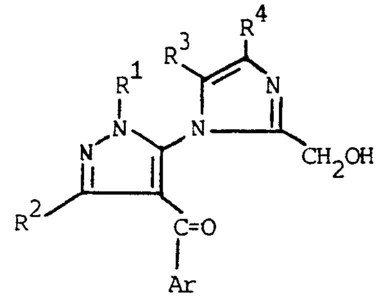

par une amine de formule $R^5R^6NH$, dans laquelle $R^1$, $R^2$, $R^3$, $R^4$, $R^5$, $R^6$ et Ar sont tels que définis ci-dessus, ce déplacement étant obtenu en transformant le groupe hydroxyle en un ester qui se déplace facilement par un nucléophile, et à faire réagir l'ester avec un excès de l'amine appropriée de formule $R^5R^6NH$.

2. Un procédé de préparation d'un composé de formule générale I, tel que défini dans la revendication 1, dans laquelle $R^5$ et $R^6$ représentent des atomes d'hydrogène, ce procédé consistant à hydrolyser, dans des conditions acides aqueuses, un composé de formule:

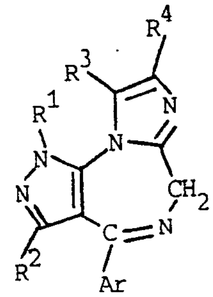

dans laquelle $R^1$, $R^2$, $R^3$, $R^4$ et Ar sont tels que définis dans la revendication 1.

3. Un procédé de préparation d'un composé de formule générale I, tel que défini dans la revendication 1, dans laquelle $R^6$ représente un atome d'hydrogène et $R^5$ représente autre chose qu'un atome d'hydrogène, ce procédé consistant à faire réagir un composé de formule:

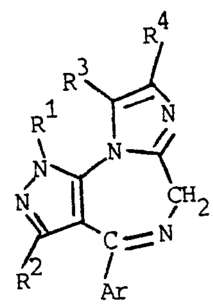

avec un excès d'un aldéhyde de formule $R^5CHO$ en présence d'acide formique, dans laquelle $R^1$, $R^2$, $R^3$, $R^4$, $R^5$ et Ar sont tels que définis dans la revendication 1.

4. Un procédé selon la revendication 1, 2 ou 3, dans lequel:

$R^1$ et $R^2$ représentent chacun un radical méthyle ou un radical éthyle;

$R^2$ et $R^3$ représentent chacun un atome d'hydrogène, un radical méthyle ou un radical éthyle;

$R^5$ et $R^6$ représentent chacun un atome d'hydrogène, un radical méthyle ou un radical éthyle; ou $R^5$ et $R^6$ pris en ensemble et avec l'atome d'azote auquel ils sont attachés, forment un groupe pyrrolidino, pipéridino, pipérazino ou morpholino non substitué ou substitué par un méthyle; et

Ar représente un radical phényle substitué par un atome de chlore ou de fluor; ou un radical 2- ou 3-thiényle.

5. Un procédé selon la revendication 1 ou 2 de préparation du composé suivant: le dichlorhydrate de {5-[2-(aminométhyl)-5-méthyl-1H-imidazol-1-yl]-1,3-diméthyl-1H-pyrazol-4-yl}(2-chlorophényl)méthanone.

6. Un procédé selon la revendication 1 de préparation du composé consistant en: le dichlorhydrate de (2-chlorophényl)-{1,3-diméthyl-5-[2-(1-pyrrolidinylméthyl)-1H-imidazol-1-yl]-1H-pyrazol-4-yl}méthanone.

7. Un procédé selon la revendication 1 de préparation du composé consistant en le {5-[2-(aminométhyl)-5-méthyl-1H-imidazol-1-yl]-1,3-diméthyl-1H-pyrazol-4-yl}(2-chlorophényl)méthanone.

8. Un procédé selon la revendication 1 de préparation du composé consistant en le (2-chlorophényl)-{1,3-diméthyl-5-[4,5-diméthyl-2-[(4-méthyl-pipérazin-1-yl)-méthyl]-1H-imidazol-1-yl]-1H-pyrazol-4-yl}méthanone.

9. Un procédé de préparation d'une composition pharmaceutique de traitement de la sénilité ou de l'amnésie qui consiste à associer une quantité efficace d'un composé préparé selon l'une quelconque des revendications 1 à 8 à un support pharmaceutiquement acceptable.

10. Pour l'utilisation dans une méthode de traitement de la sénilité ou de l'amnésie, un composé préparé par un procédé selon l'une quelconque des revendications précédentes, ou une composition pharmaceutique préparée par un procédé selon la revendication 9.